(19) Europäisches Patentamt
European Patent Office
Office européen des brevets

(11) Veröffentlichungsnummer: **0 196 520**
**A1**

(12) # EUROPÄISCHE PATENTANMELDUNG

(21) Anmeldenummer: **86103449.4**

(22) Anmeldetag: **14.03.86**

(51) Int. Cl.⁴: **C 07 C 51/567**
**C 07 C 57/04**

(30) Priorität: **20.03.85 DE 3510035**

(43) Veröffentlichungstag der Anmeldung:
**08.10.86 Patentblatt 86/41**

(84) Benannte Vertragsstaaten:
**AT BE CH DE FR GB IT LI NL**

(71) Anmelder: **BASF Aktiengesellschaft**
**Carl-Bosch-Strasse 38**
**D-6700 Ludwigshafen(DE)**

(72) Erfinder: **Bott, Kaspar, Dr.**
**Rieslingweg 4**
**D-6706 Wachenheim(DE)**

(72) Erfinder: **Anderlohr, Axel, Dr.**
**Bruehler Ring 25**
**D-6800 Mannheim 81(DE)**

(72) Erfinder: **Faust, Tillmann, Dr.**
**Parkweg 4**
**D-6714 Weisenheim(DE)**

(72) Erfinder: **Guth, Josef**
**Pfarrer-Friedrich-Strasse 41**
**D-6700 Ludwigshafen(DE)**

(54) **Verfahren zur kontinuierlichen Herstellung von Carbonsäureanhydriden.**

(57) Verfahren zur kontinuierlichen Herstellung von Carbonsäureanhydriden der allgemeinen Formel I,

```
O   O
||  ||
R–C–O–C–R
```
(I)

in der R einen gesättigten oder einen ungesättigten organischen Rest mit 2 bis 12 C-Atomen darstellt, durch säurekatalysierte Umanhydridisierung von Acetanhydrid mit einer Carbonsäure der allgemeinen Formel II,

$R\text{-}CO_2H$ (II)

in der R die oben angegebene Bedeutung hat, das dadurch gekennzeichnet ist, daß man

a) die Umanhydridisierungsreaktion im Mittelteil $K_M$ einer Destillations-kolonne K vornimmt, wobei man den einen Ausgangsstoff in den unteren Bereich und den anderen Ausgangsstoff in den oberen Beriech von $K_M$ leitet,
b) den sauren Katalysator in den Oberteil $K_O$ von K einführt,
c) am Kopf der Kolonne K die Essigsäure oder ein Gemisch von Essigsäure und Acetanhydrid entnimmt,
d) das Anhydrid I aus dem unteren Bereich des Unterteils $K_U$ von K dampfförmig oder flüssig abführt und
e) den Katalysator auf übliche Weise entweder abtrennt oder nach $K_O$ zurückleitet.

EP 0 196 520 A1

Verfahren zur kontinuierlichen Herstellung von Carbonsäureanhydriden

Die vorliegende Erfindung betrifft ein Verfahren zur kontinuierlichen Herstellung von Carbonsäureanhydriden der allgemeinen Formel I,

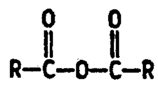 (I)

in der R einen gesättigten oder einen ungesättigten organischen Rest mit 2 bis 12 C-Atomen darstellt, durch säurekatalysierte Umanhydridisierung von Acetanhydrid mit einer Carbonsäure der allgemeinen Formel II,

$R{-}CO_2H$ (II)

in der R die oben angegebene Bedeutung hat.

Die Umsetzung erfolgt hierbei nach folgendem Reaktionsschema:

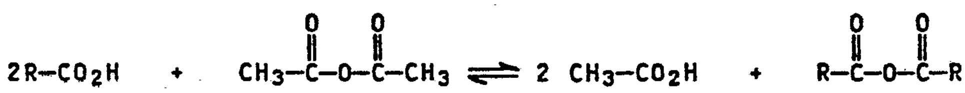

II I

Dieses Verfahren der "Umanhydridisierung" ist, sieht man von der erfindungsgemäßen Verbesserung ab, allgemein bekannt und vielfach in der Literatur beschrieben, so beispielsweise in der Zeitschrift "Journal of General Chemistry of the USSR", englische Textausgabe, Jahr 1956, Seite 1275.

Bei dem dort beschriebenen Verfahren zur Herstellung von Acrylsäureanhydrid und Methacrylsäureanhydrid wirkt sich sehr nachteilig der Zwang aus, einen Teil der Ausgangsstoffe wegen des unvollständigen Umsatzes in die Reaktion zurückführen zu müssen. Technische Verfahren aber, die mit der Rückführung von Stoffströmen belastet sind, haben im allgemeinen einen höheren Energiebedarf und benötigen größere Reaktionsapparaturen. Sie besitzen außerdem den Nachteil, daß thermisch nicht stark belastbare Stoffe wie Acrylsäureanhydrid oder Methacrylsäureanhydrid unter diesen Bedingungen leichter polymerisieren.

Der Erfindung liegt daher die Aufgabe zugrunde, eine Methode zur Herstellung von Carbonsäureanhydriden, insbesondere von Acrylsäureanhydrid und Methacrylsäureanhydrid zu entwickeln, bei der sich die Einsatzstoffe möglichst vollständig im Sinne des angeführten Reaktionsschemas umsetzen lassen.

Diese Aufgabe wird bei einem Verfahren zur Herstellung von Carbonsäureanhydriden der allgemeinen Formel I erfindungsgemäß dadurch gelöst, daß man

a) die Umanhydridisierungsreaktion im Mittelteil $K_M$ einer Destillationskolonne K vornimmt, wobei man den einen Ausgangsstoff in den unteren Bereich und den anderen Ausgangsstoff in den oberen Bereich von $K_M$ leitet,

b) den sauren Katalysator in den Oberteil $K_O$ von K einführt,

c) am Kopf der Kolonne K die Essigsäure oder ein Gemisch von Essigsäure und Acetanhydrid entnimmt,

d) das Anhydrid I aus dem unteren Bereich des Unterteils $K_U$ von K dampfförmig oder flüssig abführt und

e) den Katalysator auf übliche Weise entfernt oder nach $K_O$ zurückleitet.

Wenn die Siedepunkte der Ausgangsstoffe dicht beieinander liegen, kann es vorteilhaft sein, den leichter siedenden Ausgangsstoff in den unteren Bereich und den schwerer siedenden Ausgangsstoff in den oberen Bereich von $K_M$ zu leiten.

Das erfindungsgemäße Verfahren ist in der Zeichnung dargestellt und wird im folgenden näher erläutert.

Die Umanhydridisierungsreaktion findet im wesentlichen im Mittelteil $K_M$ der Kolonne K statt, und zwar zwischen den im Gegenstrom geführten Einsatzstoffen Acetanhydrid und Carbonsäure II. Gemäß der Zeichnung leitet man das Acetanhydrid als leichter siedende Komponente im unteren Bereich und die höher siedende Carbonsäure II im oberen Bereich von $K_M$ in die Kolonne K ein.

Um die Carbonsäure II möglichst vollständig in das Anhydrid I umwandeln zu können, muß man dafür Sorge tragen, daß die nicht umgesetzten Reaktanden durch einen entsprechenden Destillationsaufwand aus dem Oberteil $K_O$ und aus dem Unterteil $K_U$ in den mittleren Bereich $K_M$ der Kolonne K zurückgelangen. Zur Erreichung eines vollständigen Umsatzes ist es außerdem vorteilhaft, das Acetanhydrid im stöchiometrischen Überschuß über die Carbonsäure II anzuwenden. Dieser Überschuß beträgt jedoch im allgemeinen nicht mehr als 0.1 bis 0.5 Mol pro Mol Carbonsäure II. Setzt man Acetanhydrid im stöchiometrischen Überschuß über Carbonsäure II ein, so fällt am Kolonnenkopf eine Mischung aus Essigsäure und Acetanhydrid

als Destillat an. Aus dem unteren Bereich des Unterteils $K_U$ der Kolonne K entnimmt man das gewünschte Anhydrid I im flüssigen oder gasförmigen Zustand. Als saure Katalysatoren verwendet man vorzugsweise Schwefelsäure, aliphatische bzw. aromatische Sulfonsäuren oder Phosphorsäure, wobei die Menge des Katalysators zweckmäßigerweise 0.1 bis 2 Mol-%, bezogen auf die eingesetzte Carbonsäure II, beträgt. Der Katalysator kann als entsprechende Lösung in der Carbonsäure II in die Kolonne eingebracht werden. Da auch ein Teil der Carbonsäure II und des Acetanhydrids in den Kolonnenteil $K_O$ gelangt, kann hier ebenfalls eine Umanhydridisierung im geringen Umfang stattfinden. Aus diesem Grunde empfiehlt es sich, den Katalysator im oberen Bereich von $K_O$ zuzuführen. Für Versuchszwecke genügt es jedoch meistens, den Katalysator zusammen mit der Carbonsäure II in den Oberteil von $K_M$ zu geben.

Entnimmt man das Anhydrid I dampfförmig aus $K_U$, so sammelt sich im Sumpf der Kolonne K eine katalysatorhaltige Lösung an, die in den Kreislauf zurückgeführt werden kann. Im Falle einer flüssigen Entnahme des Anhydrids I ist es auch möglich, dieses in einer Seitenkolonne destillativ vom Katalysator zu befreien.

Das erfindungsgemäße Umanhydridisierungsverfahren eignet sich zur Herstellung einer Vielzahl von Anhydriden I, soweit diese und die korrespondierenden Carbonsäuren II unter den sauren Reaktionsbedingungen beständig sind. Die Reste R in Anhydriden I und Carbonsäuren II können Alkenylgruppen, Alkylgruppen oder Cycloalkylgruppen darstellen. Die vorliegende Methode ist wegen der kurzen Produkt-Verweilzeiten in der Kolonne vor allem wichtig für die Gewinnung von ungesättigten Anhydriden wie Acrylsäure- oder Methacrylsäureanhydrid, die bei Temperaturbelastung leicht polymerisieren können. Diese Anhydride besitzen nämlich eine besondere wirtschaftliche Bedeutung, weil sich aus ihnen eine große Zahl von sonst schwer zugänglichen Acrylsäurederivaten darstellen läßt, die zu Polymerisaten oder Copolymerisaten mit hervorstechenden Produkteigenschaften weiter verarbeitet werden können.

Das Herzstück der Kolonne K ist deren Mittelteil $K_M$, in welchem die Umanhydridisierungsreaktion vorwiegend abläuft.

Dieser Kolonnenteil, der im allgemeinen 3 bis 10 theoretische Trennstufen aufweisen soll, ist vorzugsweise als Glocken-, Ventil- oder Siebbodenkolonne ausgebildet, da diese Bauarten die Einstellung höherer Verweilzeiten, wie sie für die Einstellung der chemischen Gleichgewichte förderlich sind, gestatten.

Vielfach reichen aber auch Füllkörperkolonnen für die Zwecke des Kolonnenteils $K_M$ aus. Der obere Kolonnenteil $K_O$ sowie der untere Kolonnenteil $K_U$ haben dagegen die Funktion normaler Stofftrennungen und können sowohl aus Füllkörperkolonnen als auch aus Glocken-, Sieb- und Ventilbödenkolonnen bestehen. Im allgemeinen sollen $K_O$ und $K_U$ 3 bis 10 theoretische Böden aufweisen.

Prinzipiell kann man das erfindungsgemäße Umanhydridisierungsverfahren bei Normaldruck oder schwach erhöhtem Druck durchführen. Bei der Herstellung von Substanzen wie Acrylsäure- oder Methacrylsäureanhydrid, die bei thermischer Belastung leicht polymerisieren können, ist es vorteilhafter, die Kolonne K bei vermindertem Druck (10 bis 100 mbar) zu betreiben.

Beispiel

Herstellung von Methacrylsäureanhydrid

Für die Herstellung von Methacrylsäureanhydrid durch Umsetzung von Methacrylsäure mit Acetanhydrid wurde eine Versuchskolonne mit ca. 15 theoretischen Trennstufen verwendet. Diese Kolonne war 1.5 m hoch, hatte einen Innendurchmesser von 5 cm und war mit Packungen der Fa. Sulzer (BX, 150 mm) gefüllt. Der Druck am Kolonnenkopf betrug 30 mbar.

Auf der Höhe des 5. Bodens (von unten gezählt) wurden stündlich 340 g flüssiges Essigsäureanhydrid eingespeist, und auf der Höhe des 10. Bodens wurde der Kolonne stündlich eine Lösung von 6 g Methansulfonsäure in 430 g Methacrylsäure zugeführt. Der Mittelteil $K_M$ der Kolonne war somit durch die Böden 5 bis 10 definiert.

Am Kolonnenkopf fiel bei einem Rücklaufverhältnis von 5 stündlich ein Gemisch aus 300 g Essigsäure und 85 g Acetanhydrid an.

Am unteren Kolonnenende wurden stündlich 385 g Methacrylsäureanhydrid mit dem eingesetzten Katalysator entnommen. Ohne Berücksichtigung des Katalysatorgehalts besitzt das so gewonnene Methacrylsäureanhydrid eine Reinheit von 99.4 % (GC-Analyse).

Patentansprüche

1. Verfahren zur kontinuierlichen Herstellung von Carbonsäureanhydriden der allgemeinen Formel I,

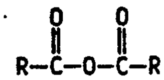 (I)

in der R einen gesättigten oder einen ungesättigten organischen Rest mit 2 bis 12 C-Atomen darstellt, durch säurekatalysierte Umanhydridisierung von Acetanhydrid mit einer Carbonsäure der allgemeinen Formel II,

$R-CO_2H$ (II)

in der R die oben angegebene Bedeutung hat, dadurch gekennzeichnet, daß man

a) die Umanhydridisierungsreaktion im Mittelteil $K_M$ einer Destillationskolonne K vornimmt, wobei man den einen Ausgangsstoff in den unteren Bereich und den anderen Ausgangsstoff in den oberen Bereich von $K_M$ leitet,

b) den sauren Katalysator in den Oberteil $K_O$ von K einführt,

c) am Kopf der Kolonne K die Essigsäure oder ein Gemisch von Essigsäure und Acetanhydrid entnimmt,

d) das Anhydrid I aus dem unteren Bereich des Unterteils $K_U$ von K dampfförmig oder flüssig abführt und

e) den Katalysator auf übliche Weise entweder abtrennt oder nach $K_O$ zurückleitet.

2. Verfahren nach Anspruch 1, dadurch gekennzeichnet, daß man den leichter siedenden Ausgangsstoff in den unteren Bereich und den schwerer siedenden Ausgangsstoff in den oberen Bereich von $K_M$ leitet.

Zeichn.

1/1

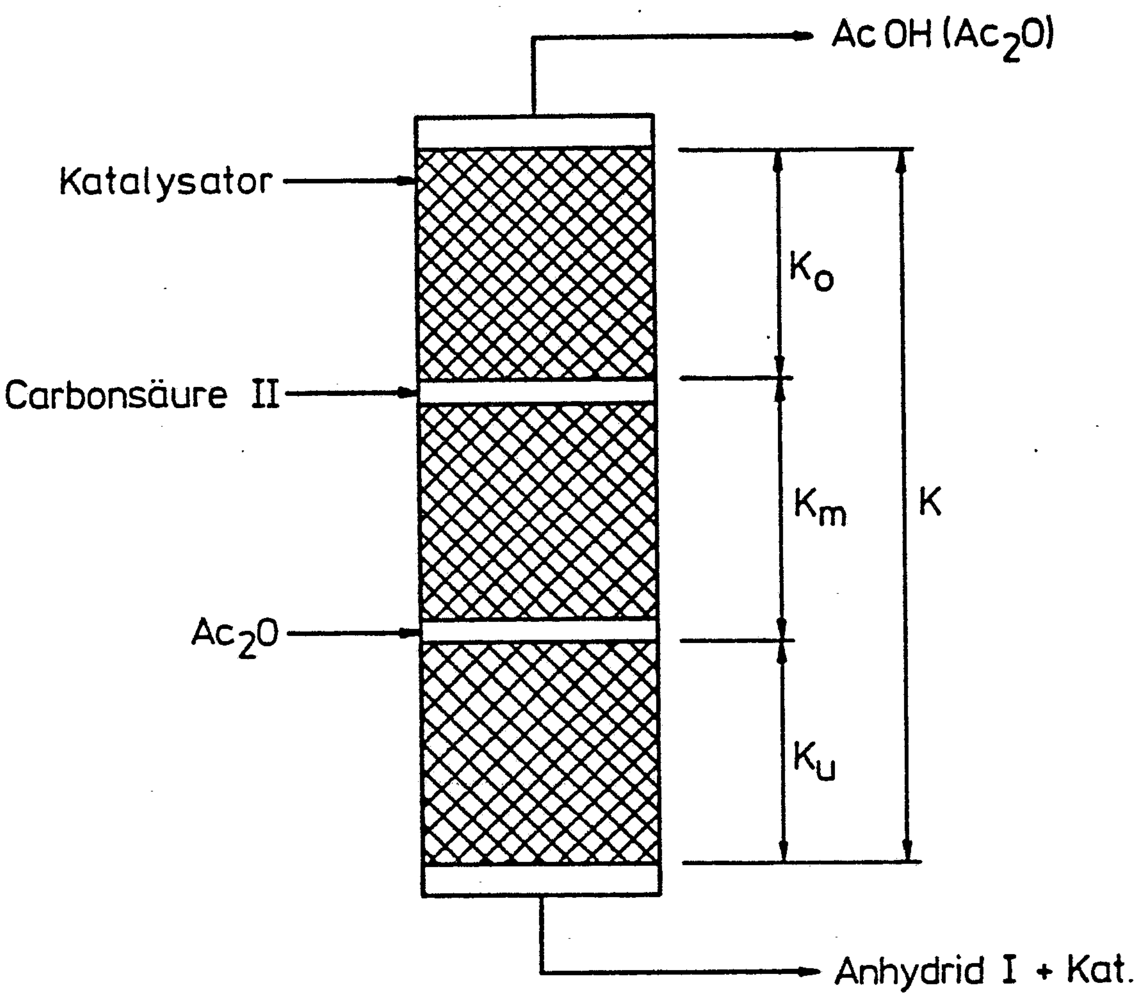
AcOH ($Ac_2O$)
Katalysator
Carbonsäure II
$Ac_2O$
$K_o$
$K_m$
$K_u$
K
Anhydrid I + Kat.

0196520

Europäisches Patentamt

# EUROPÄISCHER RECHERCHENBERICHT

Nummer der Anmeldung

EP 86 10 3449

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| A | EP-A-0 004 641 (BAYER AG)<br>* Ansprüche 1-6 *<br>----- | 1-2 | C 07 C 51/567<br>C 07 C 57/04 |
| | | | RECHERCHIERTE SACHGEBIETE (Int. Cl.4) |
| | | | C 07 C 57/00<br>C 07 C 51/00 |

Der vorliegende Recherchenbericht wurde für alle Patentansprüche erstellt.

| Recherchenort | Abschlußdatum der Recherche | Prüfer |
|---|---|---|
| DEN HAAG | 26-06-1986 | KLAG M.J. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82